# EUROPÄISCHE PATENTSCHRIFT

(11) **EP 1 756 180 B1**
(45) Veröffentlichungstag und Bekanntmachung des Hinweises auf die Patenterteilung: **09.01.2008**
(21) Anmeldenummer: 05742889.8
(22) Anmeldetag: 18.05.2005
(51) Int. Cl.: C08F 218/08, C08F 263/04, C09D 131/04

(54) **NICHTBLOCKENDE FESTHARZE VON VINYLESTER-MISCHPOLYMERISATEN**
NON-BLOCKING SOLID RESINS OF VINYL ESTER MIXED POLYMERS
RESINES SOLIDES NON COLLANTES A BASE DE COPOLYMERES DE VINYLESTER

(30) Priorität: 01.06.2004 DE 102004026608
(43) Veröffentlichungstag der Anmeldung: 28.02.2007
(73) Patentinhaber: Wacker Polymer Systems GmbH & Co. KG, 84489 Burghausen (DE)
(72) Erfinder: STARK, Kurt, 91365 Weilersbach (DE); LUMPP, Andreas Rua Vicente Oropallo, 130, 05351-025 Sao Paulo, S.P. (BR); HÖGL, Christian, 84367 Reut (DE)
(74) Vertreter: Schuderer, Michael
(86) Internationale Anmeldenummer: PCT/EP2005/005413
(87) Internationale Veröffentlichungsnummer: WO 2005/118660

(56) Entgegenhaltungen:
- EP-A- 0 381 122
- DE-A1- 4 142 104
- US-B1- 6 531 185

## Beschreibung

Die Erfindung betrifft nichtblockende Festharze von Vinylester-Mischpolymerisaten, Verfahren zu deren Herstellung sowie deren Verwendung.

Festharze von Vinylesterpolymeren oder insbesondere von Vinylester-Ethylen-Copolymeren neigen häufig zum Verblocken. Es ist aus der EP-A 959114 bekannt, dass die Oberflächenklebrigkeit von Vinylester-Ethylen-Copolymeren durch Copolymerisation mit Propylen verringert werden kann. Weiter ist bekannt, dass Silikone gute Release-Eigenschaften haben, das heißt eine klebrige Stoffe abweisende Oberfläche haben. Abmischungen bzw. Blends von Vinylester-Festharzen mit Silikonen haben allerdings unbefriedigende Eigenschaften: Aufgrund der Unverträglichkeit von Vinylesterpolymer und Silikon kommt es zur Phasenseparation bzw. zur Ausbildung von Silikondomänen und damit zur Trübung der Festharze. Die Ausbildung von Silikondomänen und das Vorliegen nicht angebundenen Silikons führt darüber hinaus zu Migrationseffekten. Aus der WO 03/085035 A1 ist ein Verfahren bekannt, wie nichtblockende Festharze auf der Basis von Vinylesterpolymerisaten mit Silikonanteilen erhalten werden.

Der Erfindung lag die Aufgabe zugrunde, Festharze auf Polyvinylester-Basis zur Verfügung zu stellen, welche unter Vermeidung der Copolymerisation von Silikonanteilen keine Neigung zur Verblockung aufweisen und ferner keine Phasenseparation in organischen Lösungsmitteln zeigen.

Überraschenderweise ist dies durch Copolymerisation von Vinylestern mit Comonomeren gelungen, welche zu Copolymeren mit mindestens zwei unterschiedlichen Glasübergangsstufen führen. Überraschend deshalb, da in der EP 381122 A2 beschrieben wird, dass die Copolymerisation von mit Vinylacetat inkompatiblen Comonomeren, wie Methylmethacrylat, zu keiner Verbessserung der Blockfestigkeit führt; und in der DE 4142104 A1 gelehrt wird, dass die Copolymerisation von Vinylacetat und Acrylsäure kommerziell nicht durchführbar ist, und zu Produkten mit sehr nachteiligen Eigenschaften führt, da Inhomogenitäten und Phasenseparation bei derartigen Copolymeren auftreten.

Gegenstand der Erfindung sind nichtblockende Festharze auf der Basis von Vinylester-Mischpolymerisaten, mit mindestens zwei unterschiedlichen Glasübergangsstufen, erhältlich mittels radikalischer Polymerisation in Substanz oder Lösung von
a) 50 bis 97 Gew.-%, bezogen auf die Gesamtmenge der Comonomere M₁ und M₂, von einem oder mehreren Comonomeren M₁ aus der Gruppe umfassend Vinylester von unverzweigten oder verzweigten Alkylcarbonsäuren mit 1 bis 15 C-Atomen, sowie gegebenenfalls einem oder mehreren alpha-Olefinen, wobei die Monomere M₁ zu Homopolymerisaten mit einer Glasübergangstemperatur Tg < 40°C führen, mit
b) 3 bis 50 Gew.-%, bezogen auf die Gesamtmenge der Comonomere M₁ und M₂, von einem oder mehreren Comonomeren M₂ aus der Gruppe umfassend Vinylaromaten, (Meth)acrylsäure und deren Ester oder deren Amide oder Acrylnitril, wobei die Comonomere M₂ zu Homopolymerisaten mit einer Glasübergangstemperatur Tg > 50°C führen, und
c) für die Copolymerisation der Comonomere M₁ mit den Comonomeren M₂ die Copolymerisationsparameter r₁ < 0.2 und r₂ > 2.0 betragen, und
d) die Comonomeren M₂ vor dem Start der Polymerisation ganz oder teilweise und zumindest in einer Menge von 3 Gew.-%, bezogen auf die Gesamtmenge der Comonomere M₁ und M₂, vorgelegt werden.

Bevorzugte Vinylester M₁ sind Vinylacetat, Vinylpropionat, Vinylbutyrat, Vinyl-2-ethylhexanoat, Vinyllaurat, 1-Methylvinylacetat, Vinylpivalat und Vinylester von α-verzweigten Monocarbonsäuren mit 5 bis 11 C-Atomen, beispielsweise VeoVa9^{R} oder VeoVa10^{R} (Handelsnamen der Firma Shell, (Vinylester von α-verzweigten Monocarbonsäuren mit 9 oder 10 C-Atomen)). Besonders bevorzugt ist Vinylacetat. Bevorzugte Olefine sind Ethylen und Propylen. Besonders bevorzugt ist Ethylen.

Im allgemeinen beträgt der Vinylesteranteil an den Comonomeren M₁ 70 bis 100 Gew.-%, bezogen auf das Gesamtgewicht der Comonomeren M₁. Bevorzugt wird als Comonomer M₁ Vinylacetat eingesetzt oder ein Gemisch aus Vinylacetat mit 1 bis 30 Gew.-% Ethylen, bezogen auf das Gesamtgewicht der Comonomere M₁.

Bevorzugte Comonomere M₂ sind Acrylsäure und Methacrylsäure, sowie Ester der Acrylsäure und Methacrylsäure von geradkettigen oder verzweigten Alkoholen mit 1 bis 15 C-Atomen sowie Acrylamid und Methacrylamid, wobei der Ester- oder Amidrest auch funktionelle oder geladene Gruppen enthalten kann, wobei die bevorzugten Comonomere M₂ jeweils zu Homopolymerisaten mit einer Glasübergangstemperatur Tg > 50°C führen. Geeignete funktionelle und geladene Gruppen sind Hydroxy-, Hydroxyalkyl-, Sulfonat-, Carboxyl-, Glycidyl-, Silan- und Ammoniumgruppen. Besonders bevorzugt werden Styrol, Acrylsäure, Methacrylsäure, Methylmethacrylat (MMA), Glycidylmethacrylat (GMA), Acrylamid, N,N-Dimethylacrylamid, Acrylnitril, 3-Methacryloxypropyltrimethoxysilan, Methacryloxymethyltrimethoxysilan, 2-Acrylamido-2-Methylpropansulfonsäure (AMPS) und N-Methylolacrylamid (NMA). Am meisten bevorzugt werden Acrylsäure, Methacrylsäure und Methylmethacrylat. Die Menge an Comonomer M₂ beträgt vorzugsweise 3 bis 40 Gew.-%, bezogen auf die Gesamtmenge der Comonomere M₁ und M₂. Die Copolymerisationsparameter r₁ und r₂ sind für jedes Monomerenpaar M₁ und M₂ charakteristische Größen und aus der Literatur bekannt: Beispielsweise aus J. Brandrup et al. Polymer Handbook.

Neben den Monomeren M₁ und M₂ können gegebenenfalls noch ein oder mehrere weitere Hilfsmonomere aus der Gruppe umfassend (Meth)acrylsäureester von Alkoholen mit 1 bis 15 C-Atomen, deren Homopolymere eine Tg kleiner 50°C aufweisen, Diene und Vinylhalogenide verwendet werden. Bevorzugte (Meth)acrylsäureester sind Methylacrylat, Ethylacrylat, Propylacrylat, n-, iso- und t-Butylacrylat sowie 2-Ethylhexylacrylat. Besonders bevorzugt sind Methylacrylat, n-, iso- und t-Butylacrylat und 2-Ethylhexylacrylat. Geeignete Diene sind 1,3-Butadien und Isopren. Aus der Gruppe der Vinylhalogenide werden üblicherweise Vinylchlorid, Vinylidenchlorid oder Vinylfluorid, vorzugsweise Vinylchlorid, eingesetzt.

Weitere Beispiele für Hilfsmonomere sind ethylenisch ungesättigte Mono- und Dicarbonsäuren, vorzugsweise Crotonsäure, Fumarsäure und Maleinsäure; weitere ethylenisch ungesättigte Carbonsäureamide und -nitrile, vorzugsweise N-Vinylformamid; auch cyclische Amide, die am Stickstoff eine ungesättigte Gruppe tragen, wie N-Vinylpyrrolidon; Mono- und Diester der Fumarsäure und Maleinsäure wie die Diethyl-, und Diisopropylester sowie Maleinsäureanhydrid, ethylenisch ungesättigte Sulfonsäuren bzw. deren Salze, vorzugsweise Vinylsulfonsäure. Als Hilfsmonomere geeignet sind auch kationische Monomere wie Diallyldimethylammoniumchlorid (DADMAC), 3-Trimethylammoniumpropyl(meth)acrylamidchlorid (MAPTAC) und 2-Trimethylammoniumethyl(meth)acrylatchlorid. Ferner sind als Hilfsmonomere geeignet Vinylether und Vinylketone.

Geeignete Hilfsmonomere sind auch weitere polymerisierbare Silane bzw. Merkaptosilane. Bevorzugt sind γ-Acryl- bzw. γ-Methacryloxypropyltri (alkoxy) silane, α-Methacryloxymethyltri(alkoxy)silane, γ-Methacryloxypropyl-methyldi(alkoxy)silane, Vinylalkyldi(alkoxy)silane und Vinyltri(alkoxy)silane, wobei als Alkoxygruppen beispielsweise Methoxy-, Ethoxy-, Methoxyethylen, Ethoxyethylen-, Methoxypropylenglykolether- bzw. Ethoxypropylenglykolether-Reste eingesetzt werden können. Beispiele hierfür sind Vinyltrimethoxysilan, Vinyltriethoxysilan, Vinyltripropoxysilan, Vinyltriisopropoxysilan, Vinyltris-(1-methoxy)-isopropoxysilan, Vinyltributoxysilan, Vinyltriacetoxysilan, 3-Methacryloxypropyltrimethoxysilan, 3-Methacryloxypropylmethyldimethoxysilan, Methacryloxymethyltrimethoxysilan, 3-Methacryloxypropyl-tris(2-methoxyethoxy)silan, Vinyltrichorsilan, Vinylmethyldichlorsilan, Vinyltris-(2-methoxyethoxy) silan, Trisacetoxyvinylsilan, 3-(Triethoxysilyl)propylbernsteinsäureanhydridsilan. Bevorzugt werden auch 3-Mercaptopropyltriethoxysilan, 3-Mercaptopropyltrimethoxysilan und 3-Mercaptopropylmethyldimethoxysilan.

Weitere Beispiele sind funktionalisierte Acrylate und funktionaliserte Vinyl- bzw. Allylether, insbesondere Epoxy-funktionelle wie Glycidylacrylat, Allylglycidether, Vinylglycidether; oder Hydroxyalkyl-funktionelle Acrylate wie Hydroxyethylacrylat, oder substituierte oder unsubstituierte Aminoalkylacrylate.

Weitere Beispiele sind vorvernetzende Comonomere wie mehrfach ethylenisch ungesättigte Comonomere, beispielsweise Divinyladipat, Diallylmaleat, Allylmethacrylat, Butandioldiacrylat oder Triallylcyanurat, oder nachvernetzende Comonomere, beispielsweise Acrylamidoglykolsäure (AGA), Methylacrylamidoglykolsäuremethylester (MAGME), N-Methylolacrylamid (NMA), N-Methylolmethacrylamid, N-Methylolallylcarbamat, Alkylether wie der Isobutoxyether oder Ester des N-Methylolacrylamids, des N-Methylolmethacrylamids und des N-Methylolallylcarbamats.

Der Anteil dieser Hilfsmonomere beträgt 0 bis 30 Gew.-%, bezogen auf die Gesamtmenge der Comonomere M₁ und M₂.

Die Herstellung der Festharze erfolgt nach dem Massepolymerisations- oder Lösungspolymerisations-Verfahren, vorzugsweise mittels Lösungspolymerisation. Bei der Lösungspolymerisation wird im allgemeinen in alkoholischer Lösung gearbeitet. Bevorzugte Lösungsmittel sind Methanol, Ethanol, tert.-Butanol, Ethylacetat, Aceton, Methylethylketon, Methylacetat und Isopropanol, wobei aber auch Mischungen von verschiedenen Lösungsmitteln verwendet werden können. Die Reaktion wird im allgemeinen unter Rückflußbedingungen, im allgemeinen bei einer Polymerisationstemperatur von 30°C bis 140°C, durchgeführt, um die Siedekühlung zur Abführung der Reaktionswärme zu nutzen. Dies kann bei Normaldruck als auch unter leichtem Überdruck erfolgen. Bei der Copolymerisation von gasförmigen Comonomeren wie Ethylen oder Vinylchlorid kann auch bei höheren Drucken, im allgemeinen bei 1 bis 100 bar gearbeitet werden.

Als Inititatoren werden organische Peroxide oder Azoverbindungen verwendet. Geeignet sind beispielsweise Diacylperoxide wie Dilauroylperoxid, Peroxoester wie t-Butylperoxopivalat, t-Butylperneodecanoat oder t-Butylperoxo-2-ethylhexanoat, Hydroperoxide wie t-Butylhydroperoxid, oder Peroxodicarbonate wie Diethylperoxodicarbonat oder Azoinitiatoren wie-AIBN. Die Initiatormenge beträgt im allgemeinen von 0.01 bis 5.0 Gew.-%, bezogen auf die Monomeren. Die Initiatoren können sowohl vorgelegt als auch dosiert werden. Dabei hat es sich bewährt, einen Teil der benötigten Initiatormenge vorzulegen und den Rest kontinuierlich während der Reaktion zu dosieren. Die vorgelegte Teilmenge bemisst sich dabei an der Menge, in welcher die Monomeren vorgelegt werden. Es kann von Vorteil sein, unterschiedlich zerfallende Initiatoren zu verschiedenen Zeitpunkten der Reaktion zu dosieren. Einen niedrig zerfallenden zu Beginn und einen bei höherer Temperatur sich zersetzenden Initiator am Ende der Reaktion, gegebenenfalls bei erhöhtem Druck.

Die Comonomeren M₁ können ganz oder teilweise vor dem Start der Polymerisation vorgelegt werden, wobei als Start der Polymerisation der Zeitpunkt gemeint ist, zu dem der Ansatz in Anwesenheit von Initiator auf Polymerisationstemperatur erwärmt ist. Vorzugsweise werden 5 bis 60 Gew.-% der Comonomeren M₁, bezogen auf die Gesamtmenge der Comonomere M₁ und M₂, vorgelegt und der Rest wird zudosiert. Die Comonomeren M₂ werden ganz oder teilweise und zumindest in einer Menge von 3 Gew.-%, bezogen auf die Gesamtmenge der Comonomere M₁ und M₂, vorgelegt. Vorzugsweise werden 3 bis 30 Gew.-% der Comonomeren M₂, bezogen auf die Gesamtmenge der Comonomere M₁ und M₂, vorgelegt, damit sich bei dem resultierenden Harz ein blockstabiles Verhalten ergibt. Besonders bevorzugt werden 3 bis 20 Gew.-% der Comonomeren M₂, bezogen auf die Gesamtmenge der Comonomere M₁ und M₂, vorgelegt und der Rest wird zudosiert. Am meisten bevorzugt werden 5 bis 10 Gew.-% der Comonomeren M₂, bezogen auf die Gesamtmenge der Comonomere M₁ und M₂, vorgelegt und der Rest wird zudosiert.

Die Reaktion wird mittels Temperaturerhöhung und gegebenenfalls Initiatorzugabe gestartet. Nach dem Ende der exothermen Reaktion werden vorzugsweise die restlichen freien Monomeren, der Regler und bei Bedarf das Lösungsmittel destillativ entfernt. Um einen sehr niedrigen VOC-Gehalt zu erhalten, wird die Innentemperatur auf Temperaturen zwischen 70°C bis 160°C erhöht und anschließend ein Vakuum angelegt.

Die Festharze können in fester Form oder als Lösung in organischen Lösungsmittel verwendet werden. Sie eignen sich generell als Bindemittel, z.B. insbesondere in Lacken, und zur Herstellung von Klebemittel, insbesondere von heißsiegelbaren Beschichtungen sowie Kaschiermitteln. Weitere Anwendungsgebiete sind die als Grundstoffe für Appreturen und Kaugummimassen. Die Festharze eignen sich ferner für den low profile Bereich, im sound damping Bereich (Schallschutz), oder als low shrinkage Additiv. Sie können im Coating- oder Powder-Coating-Bereich gewinnbringend eingesetzt werden, z.B. zur Beschichtung von Holz, Metallen, Kunststoffen, z.B. Folien, oder Glas. Auch für den Textilbereich sind die Harze bestens geeignet. Des weiteren können die Harze in der Kosmetik vorteilhafte Verwendung finden, wie z.B. in Haarsprays oder generell im Hairstyling Bereich.

Werden funktionelle Gruppen in das Harz eingebracht, z.B. bei Verwendung von funktionellen Monomeren wie Gylcidylmethacrylat, N-Methylolacrylamid, silanhaltigen Monomeren oder Acrylsäure, können die Harze vernetzt werden. Dabei kann das Harz selbst mit sich vernetzen, gegebenenfalls unter Zugabe geeigneter Katalysatoren, oder aber es können vernetzende Mischungen zweier verschiedener Harze gemacht werden, wobei zum Beispiel ein Harz eine Carbonsäurefunktion, das andere Harz eine Epoxidfunktion besitzt.

In allen Fällen zeichnen sich die erfindungsgemäßen Harze auf Polyvinylester-Basis durch eine sehr hohe Blockstabilität aus, was insbesondere bei Lagerung und Transport erhebliche Vorteile mit sich bringt. Befinden sich in den Harzen noch funktionelle Gruppen, wird damit zudem auch die Haftung auf bestimmten Substraten erhöht.

Die nachfolgenden Beispiele dienen zur weiteren Erläuterung der Erfindung, ohne diese in irgendeiner Weise einzuschränken:

### Beispiele:

### Vergleichsbeispiel 1:

In einem 120 Liter Rührkessel (drucklos), mit Rückflußkühler, Dosiereinrichtungen und einem Ankerrührer wurden 17.26 kg Methanol, 39.33 kg Vinylacetat und 7.87 g PPV (t-Butylperpivalat, 75 %-ig in Aliphaten; Halbwertszeit = 1 h bei 74°C) vorgelegt. Der Kessel wurde auf ca. 60°C aufgeheizt. Bei Erreichen von leichtem Rückfluß startete die Initiatordosierung (70.8 g PPV + 3.93 kg Methanol). Die Initiatordosierung lief über 4 Stunden mit einer Rate von 1000 g/h. Nach der Initiatordosierung lief der Ansatz noch 60 min auf Temperatur. Nach dieser Nachreaktionszeit wurde der Kessel aufgeheizt zur Destillation, dabei wurde alle 30 min frisches Methanol in den Kessel gegeben, das der abdestillierten Menge entsprach (Entmonomerisierung).

### Festharzanalysen:

FG: 30.2 %; Viskosität (Höppler - 10 %-ig in Ethylacetat gemäß DIN 53015): 11.32 mPas; Säurezahl (SZ, Methanol): 1.68 mg KOH/g; Restvinylacetat: 470 ppm; K-Wert 1 %-ig in Aceton: 42.9; Glasübergangsstufe Tg: 39.1°C

### Beispiel 2:

In einem 120 Liter Rührkessel (drucklos), mit Rückflußkühler, Dosiereinrichtungen und einem Ankerrührer wurden 17.40 kg Methanol, 35.68 kg Vinylacetat, 3.96 kg Acrylsäure und 7.93 g PPV (t-Butylperpivalat - 75 %-ig in Aliphaten; Halbwertszeit = 1 h bei 74°C) vorgelegt. Der Kessel wurde auf ca. 60°C aufgeheizt. Bei Erreichen von leichtem Rückfluß startete die Initiatordosierung (71.36 g PPV + 3.96 kg Methanol). Die Initiatordosierung lief über 4 Stunden mit einer Rate von 1010 g/h. Nach der Initiatordosierung lief der Ansatz noch 60 min auf Temperatur. Nach dieser Nachreaktionszeit wurde der Kessel aufgeheizt zur Destillation, dabei wurde alle 30 min frisches Methanol in den Kessel gegeben, das der abdestillierten Menge entsprach (Entmonomerisierung).

### Festharzanalysen:

FG: 44.3 %; SZ (Methanol): 84.15 mg KOH/g; Restvinylacetat: 610 ppm; K-Wert 1 %-ig in Aceton: 42.4; Glasübergangsstufen Tg₁: 40.6°C, Tg₂: 93.3°C

### Vergleichsbeispiel 3:

In einem 120 Liter Rührkessel (drucklos), mit Rückflußkühler, Dosiereinrichtungen und einem Ankerrührer wurden 17.40 kg Methanol, 7.14 kg Vinylacetat, 792.88 g Acrylsäure und 7.93 g PPV (t-Butylperpivalat - 75%ig in Aliphaten; Halbwertszeit = 1 h bei 74°C) vorgelegt. Der Kessel wurde auf ca. 60°C aufgeheizt. Bei Erreichen von leichtem Rückfluß startete die Initiatordosierung (71.36 g PPV + 3.96 kg Methanol). Die Initiatordosierung lief über 310 min mit einer Rate von 782 g/h. 10 min nach dem Start der Initiatordosierung begann die Monomerdosierung mit einer Rate von 7.93 kg/h. Die Monomerdosierung enthielt 28.54 kg Vinylacetat und 3.17 kg Acrylsäure. Die Dosierzeit der Monomerdosierung betrug 240 min. Nach der Initiatordosierung lief der Ansatz noch 60 min auf Temperatur. Nach dieser Nachreaktionszeit wurde der Kessel aufgeheizt zur Destillation, dabei wurde alle 30 min frisches Methanol in den Kessel gegeben, das der abdestillierten Menge entsprach (Entmonomerisierung).

### Festharzanalysen:

FG: 29.5 %; SZ (Methanol): 89.76 mg KOH/g; Restvinylacetat: 250 ppm; K-Wert 1 %-ig in Aceton: 47.8; Glasübergangsstufen Tg₁: 39.9°C, Tg₂ : 60.7°C

### Beispiel 4:

In einem 120 Liter Rührkessel (drucklos), mit Rückflußkühler, Dosiereinrichtungen und einem Ankerrührer wurden 17.40 kg Methanol, 17.84 kg Vinylacetat, 1.98 kg Acrylsäure und 7.93 g PPV (t-Butylperpivalat - 75 %-ig in Aliphaten; Halbwertszeit = 1 h bei 74°C) vorgelegt. Der Kessel wurde auf ca. 60°C aufgeheizt. Bei Erreichen von leichtem Rückfluß startete die Initiatordosierung (71.36 g PPV + 3.96 kg Methanol). Die Initiatordosierung lief über 310 min mit einer Rate von 782 g/h. 10 min nach dem Start der Initiatordosierung begann die Monomerdosierung mit einer Rate von 4.96 kg/h (17.84 kg Vinylacetat + 1.98 kg Acrylsäure). Die Dosierzeit der Monomerdosierung betrug 240 min. Nach der Initiatordosierung lief der Ansatz noch 60 min auf Temperatur. Nach dieser Nachreaktionszeit wurde der Kessel aufgeheizt zur Destillation, dabei wurde alle 30 min frisches Methanol in den Kessel gegeben, das der abdestillierten Menge entsprach (Entmonomerisierung).

### Festharzanalysen:

FG: 38.5 %; SZ (Methanol): 91.44 mg KOH/g; Restvinylacetat: 270 ppm; K-Wert 1 %-ig in Aceton: 40.9; Glasübergangsstufen Tg₁: 40.3°C, Tg₂: 84.6°C

### Beispiel 5:

In einem 120 Liter Rührkessel (drucklos), mit Rückflußkühler, Dosiereinrichtungen und einem Ankerrührer wurden 17.53 kg Methanol, 15.98 kg Vinylacetat, 4.00 kg Acrylsäure und 7.99 g PPV (t-Butylperpivalat - 75 %-ig in Aliphaten; Halbwertszeit = 1 h bei 74°C) vorgelegt. Der Kessel wurde auf ca. 60°C aufgeheizt. Bei Erreichen von leichtem Rückfluß startete die Initiatordosierung (71.93 g PPV + 4.00 kg Methanol). Die Initiatordosierung lief über 310 min mit einer Rate von 788 g/h. 10 min nach dem Start der Initiatordosierung begann die Monomerdosierung mit einer Rate von 5.0 kg/h (15.98 kg Vinylacetat + 4.00 kg Acrylsäure). Die Dosierzeit der Monomerdosierung betrug 240 min. Nach der Initiatordosierung lief der Ansatz noch 60 min auf Temperatur. Nach dieser Nachreaktionszeit wurde der Kessel aufgeheizt zur Destillation, dabei wurde alle 30 min frisches Methanol in den Kessel gegeben, das der abdestillierten Menge entsprach (Entmonomerisierung).

### Festharzanalysen:

FG: 41.5 %; SZ (Methanol): 95.37 mg KOH/g; Restvinylacetat: 800 ppm; K-Wert 1 %-ig in Aceton: 38.8; Glasübergangsstufen Tg₁: 41.8°C, Tg₂: 57.3°C, Tg₃: 98.0°C

### Vergleichsbeispiel 6:

Ein handelsübliches säurefunktionalisiertes Festharz der Wacker Polymer Systems mit K-Wert ca. 30 und ca. 5 Gew.-% Crotonsäure, ca. 95 Gew.-% Polyvinylacetat (Markenname: Vinnapas^{®} C305).

### Anwendungstechnische Untersuchungen:

### Blocktest:

Zur Bestimmung der Blockstabilität bzw. der Blockneigung wurden die Harze aus den Beispielen und Vergleichsbeispielen zunächst zu Pulvern mit vergleichbarer Korngröße gemahlen. Die Pulver wurden daraufhin in einen Messingbecher mit einem Innendurchmesser von 49 mm und einer Höhe von 3 cm gegeben. Es wurde so viel pulverförmiges Harz zugegeben, bis der Messingbecher zu ca. 1/3 gefüllt war, d.h. das Pulver stand in dem Becher mit einer Höhe von ca. 1 cm. Anschließend wurde ein Messingzylinder mit einer Masse von 1 kg und einem Durchmesser von 47 mm auf das Pulver (mit geebneter Oberfläche) in den Messingbecher gestellt. Die Vorrichtung wurde 1 h bei 50°C in den Trockenschrank gestellt. Nach dieser Zeit wurde die Apparatur dem Trockenschrank entnommen, und der Zylinder wurde entfernt. Das gepresste Pulver im Messingbecher wurde mit einem Spatel auf dessen Blockigkeit geprüft.
Die Beurteilung erfolgte mit dem Schulnotensystem:
1: Das Pulver ist nicht verblockt und lässt sich mit dem Spatel ohne weiteres zerteilen.
2: Das Pulver ist minimal verblockt, lässt sich mit dem Spatel noch problemlos zerteilen.
3: Das Pulver ist leicht verblockt und lässt sich mit dem Spatel mit leichtem Aufwand zerteilen.
4: Das Pulver ist mittelmässig verblockt und lässt sich mit dem Spatel mit größerem Aufwand zerteilen.
5: Das Pulver ist deutlich verblockt und lässt sich mit dem Spatel nur mit sehr großem Aufwand zerteilen.
6: Das Pulver ist komplett verblockt (plastifiziert) und lässt sich mit dem Spatel auch mit größtem Aufwand nicht mehr zerteilen.

**Tabelle 1: Bedingungen Blocktest: 50°C/1h/1kg (Pulver)**

| Beispiel | Blocktest |
|---|---|
| | |
| Vergleichsbeispiel 1 | 6 |
| Beispiel 2 | 2 |
| Vergleichsbeispiel 3 | 5 |
| Beispiel 4 | 2 |
| Beispiel 5 | 1 |
| Vergleichsbeispiel 6 | 5 |

Ein analoges Ergebnis wurde erhalten, als oben genannter Blocktest nicht mit Pulvern, sondern mit Filmen, die aus den Harzen aus Lösung (z.B. Methanol) hergestellt wurden, durchgeführt wurde.
In diesem Fall wurden 2 Filme des Harzes mit Abmessungen ca. 2 mm x 2 mm aufeinander gelegt und in die Blockapparatur gelegt, mit 2 kg beschwert (2 Messingzylinder) und 1 h bei 55°C in den Trockenschrank gegeben. Anschließend wurden die Filme händisch auseinandergezogen.
Bewertung nach Schulnotensystem:
1: Die Filme sind nicht miteinander verbunden und lassen sich ohne weiteres voneinander trennen.
2: Die Filme sind leicht miteinander verbunden, lassen sich mit aber ohne weiteres voneinander trennen.
3: Die Filme sind merklich miteinander verbunden, lassen sich mit aber mit geringerem Aufwand voneinander trennen.
4: Die Filme sind deutlich miteinander verbunden, lassen sich mit aber mit höherem Aufwand voneinander trennen.
5: Die Filme sind sehr stark miteinander verbunden und lassen sich nur mit sehr hohem Aufwand voneinander trennen.
6: Die Filme sind komplett miteinander verbunden (verklebt) und lassen sich auch mit dem allergrößten Aufwand nicht mehr voneinander trennen.

**Tabelle 2: Bedingungen Blocktest: 55°C/ 1h/ 2kg (Filme)**

| Beispiel | Blocktest |
|---|---|
| | |
| Vergleichsbeispiel 1 | 5 |
| Beispiel 2 | 2 |
| Vergleichsbeispiel 2 | 4 |
| Beispiel 3 | 2 |
| Beispiel 4 | 1 |

Aus der Tabelle 1 geht hervor, dass die Blockstabilität der Harze in Pulverform deutlich erhöht wird, wenn man harte Monomere, hier Acrylsäure, mit dem erfindungsgemäßen Verfahren einbringt (Beispiele 2, 4 und 5). Dabei stellt sich der Effekt schon mit einer geringen Menge an hartem Monomer ab 3 bzw. hier 5 Gew.-% im Harz ein, wenn dieses in die Vorlage gegeben wird. Der Vergleich von Beispiel 2 und 4 mit Vergleichsbeispiel 3 (alle besitzen insgesamt 10 Gew.-% Acrylsäure im Harz) zeigt, dass ein blockstabiles Verhalten bei den Harzen nur dann erreicht werden kann, wenn eine höherere Menge an hartem Monomer in die Vorlage gegeben wird. So wurde bei Vergleichsbeispiel 3 mit nur 2 Gew.-% Acrylsäure (jeweils bezogen auf die Gesamtmonomermenge) in der Vorlage eine Blockbewertung von 5 erhalten (hohe Blockneigung), während bei Beispiel 4 (5 Gew.-% Acrylsäure in der Vorlage) und bei Beispiel 2 (10 Gew.-% Acrylsäure in der Vorlage) eine Blockbewertung von 2 (sehr geringe Blockneigung) erhalten wurde. Das heisst, dass trotz der gleichen Menge an hartem Monomer das Verfahren der Polymerisation (Vorlage, Dosierung) den entscheidenden Effekt auf das Blockverhalten ausübt.

Ein Harz ohne hartes Monomer weist bei den Untersuchungsbedingungen vollständige Verblockung auf (Blockbewertung 6, Vergleichsbeispiel 1). Verwendet man statt einem harten Monomer, das sehr schlecht mit Vinylacetat copolymerisiert, die gleiche Menge eines harten Monomeren, das bedeutend besser mit Vinylacetat copolymerisiert, so wird keine Verbesserung der Blockstabiltität erreicht (Vergleichsbeispiel 6 mit Crotonsäure statt Acrylsäure, Blockbewertung 5). Das bedeutet, dass neben dem Verfahren der Polymerisation auch die Copolymerisationsparameter der Comonomeren zueinander eine entscheidende Rolle bezüglich der Blockneigung spielen.

Ein analoges Ergebnis ergab sich bei den anwendungstechnischen Messungen an den Filmen der Harze aus den Beispielen und Vergleichsbeispielen. Alle erfindungsgemäßen Harze zeigten - trotzdem diese 2 oder mehrere Glasübergangsstufen Tg hatten - keine Phasenseparation in organischem Lösungsmittel oder in der Schmelze. Auch dieses Verhalten kann nur mit dem erfindungsgemäßen Polymerisationsverfahren erreicht werden.

## Patentansprüche

1. Nichtblockende Festharze auf der Basis von Vinylester-Mischpolymerisaten, mit mindestens zwei unterschiedlichen Glasübergangsstufen, erhältlich mittels radikalischer Polymerisation in Substanz oder Lösung von
a) 50 bis 97 Gew.-%, bezogen auf die Gesamtmenge der Comonomere M₁ und M₂, von einem oder mehreren Comonomeren M₁ aus der Gruppe umfassend Vinylester von unverzweigten oder verzweigten Alkylcarbonsäuren mit 1 bis 15 C-Atomen, sowie gegebenenfalls einem oder mehreren alpha-Olefinen, wobei die Monomere M₁ zu Homopolymerisaten mit einer Glasübergangstemperatur Tg < 40°C führen, mit
b) 3 bis 50 Gew.-%, bezogen auf die Gesamtmenge der Comonomere M₁ und M₂, von einem oder mehreren Comonomeren M₂ aus der Gruppe umfassend Vinylaromaten, (Meth)acrylsäure und deren Ester oder deren Amide oder Acrylnitril, wobei die Comonomere M₂ zu Homopolymerisaten mit einer Glasübergangstemperatur Tg > 50°C führen, und
c) für die Copolymerisation der Comonomere M₁ mit den Comonomeren M₂ die Copolymerisationsparameter r₁ < 0.2 und r₂ > 2.0 betragen, und
d) die Comonomeren M₂ vor dem Start der Polymerisation teilweise und zumindest in einer Menge von 3 Gew.-%, bezogen auf die Gesamtmenge der Comonomere M₁ und M₂, vorgelegt werden.

2. Verfahren zur Herstellung von nichtblockenden Festharzen auf der Basis von Vinylester-Mischpolymerisaten, mit mindestens zwei unterschiedlichen Glasübergangsstufen, mittels radikalischer Polymerisation in Substanz oder Lösung von
a) 50 bis 97 Gew.-%, bezogen auf die Gesamtmenge der Comonomere M₁ und M₂, von einem oder mehreren Comonomeren M₁ aus der Gruppe umfassend Vinylester von unverzweigten oder verzweigten Alkylcarbonsäuren mit 1 bis 15 C-Atomen, sowie gegebenenfalls einem oder mehreren alpha-Olefinen, wobei die Monomere M₁ zu Homopolymerisaten mit einer Glasübergangstemperatur Tg < 40°C führen, mit
b) 3 bis 50 Gew.-%, bezogen auf die Gesamtmenge der Comonomere M₁ und M₂, von einem oder mehreren Comonomeren M₂ aus der Gruppe umfassend Vinylaromaten, (Meth)acrylsäure und deren Ester oder deren Amide oder Acrylnitril, wobei die Comonomere M₂ zu Homopolymerisaten mit einer Glasübergangstemperatur Tg > 50°C führen, und
c) für die Copolymerisation der Comonomere M₁ mit den Comonomeren M₂ die Copolymerisationsparameter r₁ < 0.2 und r₂ > 2.0 betragen, und
d) die Comonomeren M₂ vor dem Start der Polymerisation ganz oder teilweise und zumindest in einer Menge von 3 Gew.-%, bezogen auf die Gesamtmenge der Comonomere M₁ und M₂, vorgelegt werden.

3. Verfahren nach Anspruch 2, **dadurch gekennzeichnet, dass** als Comonomer M₁ Vinylacetat eingesetzt wird, oder ein Gemisch aus Vinylacetat mit 1 bis 30 Gew.-% Ethylen, bezogen auf das Gesamtgewicht der Comonomere M₁.

4. Verfahren nach Anspruch 2 oder 3, **dadurch gekennzeichnet, dass** als Comonomer M₂ ein oder mehrere eingesetzt werden, aus der Gruppe umfassend Acrylsäure und Methacrylsäure, sowie Ester der Acrylsäure und Methacrylsäure von geradkettigen oder verzweigten Alkoholen mit 1 bis 15 C-Atomen sowie Acrylamid und Methacrylamid, wobei der Ester- oder Amidrest auch funktionelle oder geladene Gruppen enthalten kann, wobei die Comonomere M₂ jeweils zu Homopolymerisaten mit einer Glasübergangstemperatur Tg > 50°C führen.

5. Verfahren nach Anspruch 4, **dadurch gekennzeichnet, dass** als Comonomer M₂ ein oder mehrere eingesetzt werden, aus der Gruppe umfassend Styrol, Acrylsäure, Methacrylsäure, Methylmethacrylat, Glycidylmethacrylat, Acrylamid, N,N-Dimethylacrylamid, Acrylnitril, 2-Acrylamido-2-Methyl-propansulfonsäure und N-Methylolacrylamid.

6. Verfahren nach Anspruch 2 bis 5, **dadurch gekennzeichnet, dass** 5 bis 60 Gew.-% der Comonomeren M₁, bezogen auf die Gesamtmenge der Comonomere M₁ und M₂, vorgelegt und der Rest zudosiert wird.

7. Verfahren nach Anspruch 2 bis 6, **dadurch gekennzeichnet, dass** 3 bis 30 Gew.-% der Comonomeren M₂, bezogen auf die Gesamtmenge der Comonomere M₁ und M₂, vorgelegt und der Rest wird zudosiert.

8. Verwendung der nichtblockenden Festharze gemäß Anspruch 1 und der Verfahrensprodukte gemäß Anspruch 2 bis 7 als Bindemittel in Lacken und zur Herstellung von Klebemitteln.

9. Verwendung der nichtblockenden Festharze gemäß Anspruch 1 und der Verfahrensprodukte gemäß Anspruch 2 bis 7 als Grundstoffe für Appreturen und Kaugummimassen.

10. Verwendung der nichtblockenden Festharze gemäß Anspruch 1 und der Verfahrensprodukte gemäß Anspruch 2 bis 7 als Additive für den low profile Bereich, als Additive für den Schallschutz und als low shrinkage Additiv.

11. Verwendung der nichtblockenden Festharze gemäß Anspruch 1 und der Verfahrensprodukte gemäß Anspruch 2 bis 7 im Coating- und Powder-Coating-Bereich zur Beschichtung von Holz, Metallen, Kunststoffen, und Glas.

12. Verwendung der nichtblockenden Festharze gemäß Anspruch 1 und der Verfahrensprodukte gemäß Anspruch 2 bis 7 im Textilbereich.

13. Verwendung der nichtblockenden Festharze gemäß Anspruch 1 und der Verfahrensprodukte gemäß Anspruch 2 bis 7 in der Kosmetik.

## Claims

1. Non-blocking solid resins based on vinyl ester copolymers, having at least two different glass transition stages, obtainable by means of free-radical polymerization in bulk or solution of
a) 50% to 97% by weight, based on the total amount of the comonomers M₁ and M₂, of one or more comonomers M₁ from the group consisting of vinyl esters of branched or of unbranched alkylcarboxylic acids having 1 to 15 carbon atoms, and, if desired, one or more alpha-olefins, the monomers M₁ leading to homopolymers having a glass transition temperature Tg < 40°C, with
b) 3% to 50% by weight, based on the total amount of the comonomers M₁ and M₂, of one or more comonomers M₂ from the group consisting of vinylaromatics, (meth)acrylic acid and esters thereof or amides thereof or acrylonitrile, the comonomers M₂ leading to homopolymers having a glass transition temperature Tg > 50°C, and
c) for the copolymerization of the comonomers M₁ with the comonomers M₂ the copolymerization parameters amount to r₁ < 0.2 and r₂ > 2.0, and
d) some of the comonomers M₂, and an amount of at least 3% by weight, based on the total amount of the comonomers M₁ and M₂, are introduced before the start of the polymerization.

2. Process for preparing non-blocking solid resins based on vinyl ester copolymers, having at least two different glass transition stages, by means of free-radical polymerization in bulk or solution of
a) 50% to 97% by weight, based on the total amount of the comonomers M₁ and M₂, of one or more comonomers M₁ from the group consisting of vinyl esters of branched or of unbranched alkylcarboxylic acids having 1 to 15 carbon atoms, and, if desired, one or more alpha-olefins, the monomers M₁ leading to homopolymers having a glass transition temperature Tg < 40°C, with
b) 3% to 50% by weight, based on the total amount of the comonomers M₁ and M₂, of one or more comonomers M₂ from the group consisting of vinylaromatics, (meth)acrylic acid and esters thereof or amides thereof or acrylonitrile, the comonomers M₂ leading to homopolymers having a glass transition temperature Tg > 50°C, and
c) for the copolymerization of the comonomers M₁ with the comonomers M₂ the copolymerization parameters amount to r₁ < 0.2 and r₂ > 2.0, and
d) some or all of the comonomers M₂, and an amount of at least 3% by weight, based on the total amount of the comonomers M₁ and M₂, are introduced before the start of the polymerization.

3. Process according to Claim 2, **characterized in that** as comonomer M₁ vinyl acetate or a mixture of vinyl acetate with 1% to 30% by weight of ethylene, based on the total weight of the comonomers M₁, is used.

4. Process according to Claim 2 or 3, **characterized in that** as comonomer M₂ one or more are used from the group consisting of acrylic acid and methacrylic acid, and also esters of acrylic and methacrylic acid with straight-chain or branched alcohols having 1 to 15 carbon atoms, and also acrylamide and methacrylamide, it also being possible for the ester or amide radical to contain functional or charged groups, the comonomers M₂ each leading to homopolymers having a glass transition temperature Tg > 50°C.

5. Process according to Claim 4, **characterized in that** as comonomer M₂ one or more are used from the group consisting of styrene, acrylic acid, methacrylic acid, methyl methacrylate, glycidyl methacrylate, acrylamide, N,N-dimethylacrylamide, acrylonitrile, 2-acrylamido-2-methylpropanesulphonic acid and N-methylolacrylamide.

6. Process according to Claim 2 to 5, **characterized in that** 5% to 60% by weight of the comonomers M₁, based on the total amount of the comonomers M₁ and M₂, is included in the initial charge and the remainder is metered in.

7. Process according to Claim 2 to 6, **characterized in that** 3% to 30% by weight of the comonomers M₂, based on the total amount of the comonomers M₁ and M₂, is included in the initial charge and the remainder is metered in.

8. Use of the non-blocking solid resins according to Claim 1 and of the process products according to Claim 2 to 7 as binders in coating materials and for preparing adhesives.

9. Use of the non-blocking solid resins according to Claim 1 and of the process products according to Claim 2 to 7 as base materials for finishing agents and chewing-gum masses.

10. Use of the non-blocking solid resins according to Claim 1 and of the process products according to Claim 2 to 7 as additives for the low-profile sector, as additives for soundproofing and as low-shrinkage additives.

11. Use of the non-blocking solid resins according to Claim 1 and of the process products according to Claim 2 to 7 in the coating and powder-coating sector for the coating of wood, metals, plastics and glass.

12. Use of the non-blocking solid resins according to Claim 1 and of the process products according to Claim 2 to 7 in the textile sector.

13. Use of the non-blocking solid resins according to Claim 1 and of the process products according to Claim 2 to 7 in cosmetology.

## Revendications

1. Résines solides non collantes à base de copolymères d'ester de vinyle, présentant au moins deux stades de transition vitreuse différents, pouvant être obtenues par polymérisation radicalaire en masse ou en solution de
a) 50 à 97% en poids, par rapport à la quantité totale des comonomères M₁ et M₂, d'un ou de plusieurs comonomères M₁ du groupe comprenant les esters de vinyle d'acides alkylcarboxyliques non ramifiés ou ramifiés comprenant 1 à 15 atomes de carbone, ainsi que le cas échéant d'une ou de plusieurs alpha-oléfines, les monomères M₁ conduisant à des homopolymères présentant une température de transition vitreuse Tg < 40°C, avec
b) 3 à 50% en poids, par rapport à la quantité totale des comonomères M₁ et M₂, d'un ou de plusieurs comonomères M₂ du groupe comprenant les aromatiques de vinyle, l'acide (méth)acrylique et ses esters ou ses amides ou l'acrylonitrile, les monomères M₂ conduisant à des homopolymères présentant une température de transition vitreuse Tg >50°C, et
c) pour la copolymérisation des comonomères M₁ avec les comonomères M₂, les paramètres de copolymérisation étant r₁ < 0,2 et r₂ > 2,0, et
d) les comonomères M₂ étant disposés, avant le début de la polymérisation, en partie et au moins en une quantité de 3% en poids, par rapport à la quantité totale des comonomères M₁ et M₂.

2. Procédé pour la préparation de résines solides non collantes à base de copolymères d'ester de vinyle, présentant au moins deux stades de transition vitreuse différents, au moyen d'une polymérisation radicalaire en masse ou en solution de
a) 50 à 97% en poids, par rapport à la quantité totale des comonomères M₁ et M₂, d'un ou de plusieurs comonomères M₁ du groupe comprenant les esters de vinyle d'acides alkylcarboxyliques non ramifiés ou ramifiés comprenant 1 à 15 atomes de carbone, ainsi que le cas échéant d'une ou de plusieurs alpha-oléfines, les monomères M₁ conduisant à des homopolymères présentant une température de transition vitreuse Tg < 40°C, avec
b) 3 à 50% en poids, par rapport à la quantité totale des comonomères M₁ et M₂, d'un ou de plusieurs comonomères M₂ du groupe comprenant les aromatiques de vinyle, l'acide (méth)acrylique et ses esters ou ses amides ou l'acrylonitrile, les monomères M₂ conduisant à des homopolymères présentant une température de transition vitreuse Tg >50°C, et
c) pour la copolymérisation des comonomères M₁ avec les comonomères M₂, les paramètres de copolymérisation étant r₁ < 0,2 et r₂ > 2,0, et
d) les comonomères M₂ étant disposés, avant le début de la polymérisation, totalement ou en partie et au moins en une quantité de 3% en poids, par rapport à la quantité totale des comonomères M₁ et M₂.

3. Procédé selon la revendication 2, **caractérisé en ce qu'**on utilise comme comonomère M₁ de l'acétate de vinyle ou un mélange d'acétate de vinyle avec 1 à 30% en poids d'éthylène, par rapport au poids total des comonomères M₁.

4. Procédé selon la revendication 2 ou 3, **caractérisé en ce qu'**on utilise comme comonomère M₂ un ou plusieurs comonomères du groupe comprenant l'acide acrylique et méthacrylique ainsi que les esters de l'acide acrylique et méthacrylique d'alcools linéaires ou ramifiés, comprenant 1 à 15 atomes de carbone ainsi que l'acrylamide et le méthacrylamide, le radical ester ou amide pouvant également contenir des groupes fonctionnels ou chargés, les comonomères M₂ conduisant à chaque fois à des homopolymères présentant une température de transition vitreuse Tg > 50°C.

5. Procédé selon la revendication 4, **caractérisé en ce qu'**on utilise comme comonomère M₂ un ou plusieurs comonomères du groupe comprenant le styrène, l'acide acrylique, l'acide méthacrylique, le méthacrylate de méthyle, le méthacrylate de glycidyle, l'acrylamide, le N,N-diméthylacrylamide, l'acrylonitrile, l'acide 2-acrylamido-2-méthylpropanesulfonique et le N-méthylolacrylamide.

6. Procédé selon la revendication 2 à 5, **caractérisé en ce qu'**on dispose au préalable 5 à 60% en poids des comonomères M₁, par rapport à la quantité totale des comonomères M₁ et M₂, et le reste est ajouté en dosant.

7. Procédé selon la revendication 2 à 6, **caractérisé en ce qu'**on dispose au préalable 3 à 30% en poids des comonomères M₂, par rapport à la quantité totale des comonomères M₁ et M₂, et le reste est ajouté en dosant.

8. Utilisation des résines solides non collantes selon la revendication 1 et des produits de procédé selon la revendication 2 à 7 comme liants dans des laques et pour la production d'adhésifs.

9. Utilisation des résines solides non collantes selon la revendication 1 et des produits de procédé selon la revendication 2 à 7 comme matières de base pour des apprêts et des masses pour chewing-gum.

10. Utilisation des résines solides non collantes selon la revendication 1 et des produits de procédé selon la revendication 2 à 7 comme additifs pour le domaine des résines à dimensions stables (low profile), comme additifs pour la protection contre le bruit et comme additif de faible retrait (low shrinkage).

11. Utilisation des résines solides non collantes selon la revendication 1 et des produits de procédé selon la revendication 2 à 7 dans le domaine des revêtements et des revêtements de poudre pour le revêtement de bois, de métaux, de matériaux synthétiques et de verre.

12. Utilisation des résines solides non collantes selon la revendication 1 et des produits de procédé selon la revendication 2 à 7 dans le domaine des textiles.

13. Utilisation des résines solides non collantes selon la revendication 1 et des produits de procédé selon la revendication 2 à 7 en cosmétique.
